Europäisches Patentamt

European Patent Office

Office européen des brevets

(19)

(11) Numéro de publication : **0 438 359 B1**

(12) **FASCICULE DE BREVET EUROPEEN**

(45) Date de publication du fascicule du brevet :
30.03.94 Bulletin 94/13

(51) Int. Cl.⁵ : **A61K 9/16**

(21) Numéro de dépôt : **91400106.0**

(22) Date de dépôt : **18.01.91**

(54) **Procédé de préparation de médicaments sous forme de perles.**

Le dossier contient des informations techniques présentées postérieurement au dépôt de la demande et ne figurant pas dans le présent fascicule.

(30) Priorité : **19.01.90 FR 9000623**

(43) Date de publication de la demande :
**24.07.91 Bulletin 91/30**

(45) Mention de la délivrance du brevet :
**30.03.94 Bulletin 94/13**

(84) Etats contractants désignés :
**AT BE CH DE DK ES FR GB GR IT LI LU NL SE**

(56) Documents cités :
**DE-A- 2 725 849**
**DE-A- 2 725 924**
**FR-A- 1 302 362**
**FR-A- 2 266 692**
**US-A- 4 389 356**
**The Merck Index, Eleventh Edition (1989),**
**pages 776, 836, 921**

(73) Titulaire : **RHONE-POULENC RORER S.A.**
**20, avenue Raymond Aron**
**F-92160 Antony (FR)**

(72) Inventeur : **Deleuil, Michel**
**1 avenue de la Résidence**
**F-92160 Antony (FR)**
Inventeur : **Labourt-Ibarre, Pierre**
**107 rue Garibaldi**
**F-69006 Lyon (FR)**
Inventeur : **Rona, Robert**
**3 Allée des Chênes**
**F-78100 Saint-Germain-En-Laye (FR)**
Inventeur : **Statiotis, Eraclis**
**3 Impasse des Hévéas**
**F-38280 Villette d'Anthon (FR)**

(74) Mandataire : **Le Pennec, Magali et al**
**RHONE-POULENC RORER SA, Direction des**
**Brevets, 20 Avenue Raymond Aron**
**F-92165 Antony Cédex (FR)**

## Description

La présente invention concerne une nouvelle présentation de principes actifs pharmaceutiques sous forme de perles ainsi que leur procédé de préparation. Elle concerne plus particulièrement la mise sous forme de perles de principes actifs médicamenteux qui présentent un phénomène de surfusion.

Les composés chimiques mis sous forme de perles sont plus connus dans l'art antérieur sous le terme de "prills". Ils concernent pour la plupart des produits minéraux ne présentant aucun phénomène de dégradation ni à la chaleur ni à l'humidité. La mise sous forme de perles est simplement réalisée pour une commodité d'emploi car les perles présentent une distribution régulière quant au diamètre et à la forme des particules.

La fabrication de ces perles est décrite notamment dans les brevets EP 277 508, US 4 525 198, US 4 389 356. Les procédés de préparation de ces perles consistent dans une première étape à faire fondre le composé que l'on doit formuler puis à le conduire à travers une filière ou une plaque perforée qui est soumise à sa base à des flux qui permettent de former des perles ou des billes qui tombent dans une tour à contre courant avec un gaz ou de l'air permettant ainsi la solidification des billes qui n'adhèrent pas aux parois de la tour.

Ce procédé est largement utilisé pour la mise en forme des engrais (urée, azoté, phosphoré,...) car ces produits peuvent être soumis à un chauffage violent pour les faire fondre et à un refroidissement rapide pour assurer leur solidification. En plus, la régularité de la dimension des perles (environ quelques millimètres) n'est pas une condition aussi draconienne dans l'industrie des engrais que dans l'industrie pharmaceutique où l'on désire des dimensions de perles de l'ordre de quelques dizaines à quelques centaines de microns.

D'autres procédés de préparation de perles sont décrits dans les brevets DE-A-2 725 924 et FR-A-1 302 362. DE-A-2 725 924 décrit un procédé de préparation de perles par "prilling" qui consiste à fondre un excipient dont le point de fusion est inférieur à 120°C dans lequel on peut ajouter un principe actif dissous ou dispersé mais jamais fondu, on fait passer cette dispersion fondue à travers une buse vibrée et on refroidit les perles formées. FR-A-1 302 362 utilise également une dispersion fondue pour former des perles. Le principe actif n'est pas fondu et ne présente pas de phénomènes de surfusion.

Certains principes actifs présentent lors de leur fusion des phénomènes dits de "surfusion" qui retardent considérablement leur solidification même après refroidissement. Le problème qu'a cherché à résoudre la présente invention est celui de la mise sous forme de billes de ces principes actifs médicamenteux qui présentent un point de cristallisation non défini (ou plus communément un phénomène de surfusion) et qui sont donc peu favorables à la technique du "prilling" car ils ont tendance à rester sous forme huileuse ou pâteuse bien après un retour à une température inférieure à leur température de fusion. Lors du passage de ce type de composés dans une tour de prilling, on s'attendait à avoir une masse pâteuse au fond de la tour ou sur les parois. La technique qui consistait à envoyer un jet d'air très froid (-10°C à -20°C) sur le jet de substance fondue à la sortie de la buse ne pouvait permettre de résoudre le problème. En effet, lorsque des produits en surfusion sont mis en contact d'un froid important, la viscosité du liquide augmente considérablement et ralentit la cristallisation ultérieure. Il fallait donc éviter à tout prix d'introduire à la sortie de la buse un jet d'air trop froid.

La présente invention a permis de résoudre ce problème sans avoir recours à un refroidissement très intense ni à des tours de hauteur trop importante.

Elle consiste en un procédé de préparation de perles à base de principe actif pharmaceutique dans lequel on mélange ledit principe actif avec un excipient pharmaceutique favorisant la solidification du principe actif, on force le passage de la masse fondue à travers une buse qui subit une vibration, on laisse tomber les perles formées dans une tour à contre courant avec un gaz, on rassemble les perles formées dans le bas de la tour caractérisé en ce que le principe actif présente un point de cristallisation non défini et est utilisé à l'état fondu.

A cette tour de prilling est éventuellement adjoint un lit fluidisé qui permet de maintenir en fluidisation permanente les perles non encore entièrement solidifiées.

Les principes actifs ayant un point de solidification non défini qui présentent donc un phénomène de surfusion sont choisis notamment parmi :
- l'acide (benzoyl-3 phényl)-2 propionique ou kétoprofène,
- le dicarbamate de méthyl-2 propyl-2-propanediol-1,3 ou méprobamate
- l'acide (isobutyl-4 phényl)-2 propionique

Les additifs qui permettent d'induire la cristallisation du produit en surfusion sont choisis parmi les produits utilisés pour l'usage pharmaceutique tels que :
- les alcools gras tels que :
  . l'alcool cétylique,
  . l'alcool stéarilique,
- les acides gras tels que :
  . l'acide stéarique,
  . l'acide palmitique,

- les esters de glycérol tels que :
   . le palmitostéarate de glycérol,
   . le stéarate de glycérol commercialisé sous la marque PRECIROL®,
   . le béhénate de glycérol commercialisé sous la marque COMPRITOL®,
- les huiles hydrogénées telles que :
   . l'huile de ricin hydrogénée commercialisée sous la marque CUTINA HR®,
- les sels d'acides gras tels que :
   . le stéarate de magnésium ou de calcium,
- les polyols tels que :
   . le mannitol,
   . le sorbitol,
   . le xylitol,
- les cires telles que :
   . la cire blanche,
   . la cire de Carnauba,
   . la paraffine,
- les polyoxyéthylène glycols de poids moléculaire élevé,
- les polyoxyéthylène estérifiés tels que :
   . le distéarate de PEG-32,
   . le distéarate de PEG-150.

Parmi les principes actifs présentant un problème de surfusion, le problème est particulièrement important dans le cas de l'acide (benzoyl-3 phényl)-2 propionique. Les excipients qui pourront être mis en oeuvre pour favoriser la solidification de ce composé doivent bien évidemment être inertes vis-à-vis de celui-ci. Ainsi ne pourront être utilisées que les classes d'excipients suivantes :

- les acides gras et leurs sels,
- les esters du glycérol,
- les huiles hydrogénées,
- les cires,
- les polyoxyéthylèneestérifiés,

Pour l'acide (benzoyl-3 phényl)-2 propionique, on préfère utiliser les esters de glycérol en mélange avec l'acide stéarique. On préfère encore utiliser au moins 20 % d'excipient pour formuler jusqu'à 80 % de principe actif et de préférence entre 30 et 40 % d'excipient. On préfère plus particulièrement un excipient contenant au moins 20 % d'acide stéarique.

A ces additifs de cristallisation, il est parfois souhaitable d'adjoindre des polymères solubles ou dispersibles dans la masse fondue qui vont permettre une dissolution maîtrisée et modulable des perles lors de leur utilisation tels que :

- les dérivés cellulosiques (hydroxypropylcellulose, hydroxypropylméthylcellulose, hydroxyéthylcellulose, éthylcellulose, carboxyméthylcellulose),
- les résines acryliques (commercialisées sous la marque EUDRAGIT=®),
- les polyvinylacétates (commercialisées sous la marque RHODOPAS=®),
- les résines de polyalkylène (éthylène propylène), de polylactique, d'anhydride maléique, de silicone.

Certains additifs minéraux permettent, avec les additifs de cristallisation d'accélérer la solidification des principes actifs présentant ce phénomène de surfusion. On peut citer à titre d'exemple :

- les silices,
- les oxydes minéraux tels que l'oxyde de titane, de fer,
- les phosphates,
- les carbonates,
- les argiles,
- le talc.

Afin d'améliorer la dispersion du principe actif dans l'additif de cristallisation, il est parfois utile d'ajouter un agent tensioactif choisi par exemple parmi les esters de sorbitol, les polysorbates de polyoxyéthylène commercialisés sous la marque TWEEN, les glycols tels que la glycétine ou le propylène glycol.

Le procédé de préparation des perles consiste à préparer une masse fondue du ou des principes actifs avec un ou plusieurs excipients. Cette masse peut être préparée par fusion séparée des divers constituants suivie de leur mélange ou par fusion du mélange des constituants. Les éventuels composés non solubles étant additionnés à la fin de la fusion de façon à obtenir une masse homogène.

La nature des constituants de la masse fondue est choisie par l'homme de l'art de la technique considérée en fonction de la compatibilité des constituants, de la viscosité du mélange des constituants, du diamètre de la buse, de l'hydrophilie du principe actif, de la tension superficielle du principe actif, de la taille particulaire des additifs insolubles, du débit de la buse, de la température de la tour, de sa hauteur et surtout de la dimension des perles désirées, du taux de principe actif à y inclure et du temps de libération du principe actif désiré.

La libération "in vitro" et la disponibilité "in vivo" du principe actif à partir de ces perles est modifiée (prolongée, retardée ou différée) grâce à l'additif de cristallisation qui selon sa nature permet une libération du principe actif en des temps deux à vingt fois plus longs que le même principe actif conditionné de façon classique par exemple sous forme de comprimés à libération immédiate. Ainsi, ces perles permettent une prise des médicaments préparés à partir de celles-ci quotidienne au lieu des 2 ou 3 prises quotidiennes avec le médicament classique à libération immédiate.

Parmi les additifs de cristallisation permettant un effet retard, on peut citer non limitativement les acides gras, les esters de glycérol, les huiles hydrogénées, les cires et les polyoxyéthylèneglycols estérifiées.

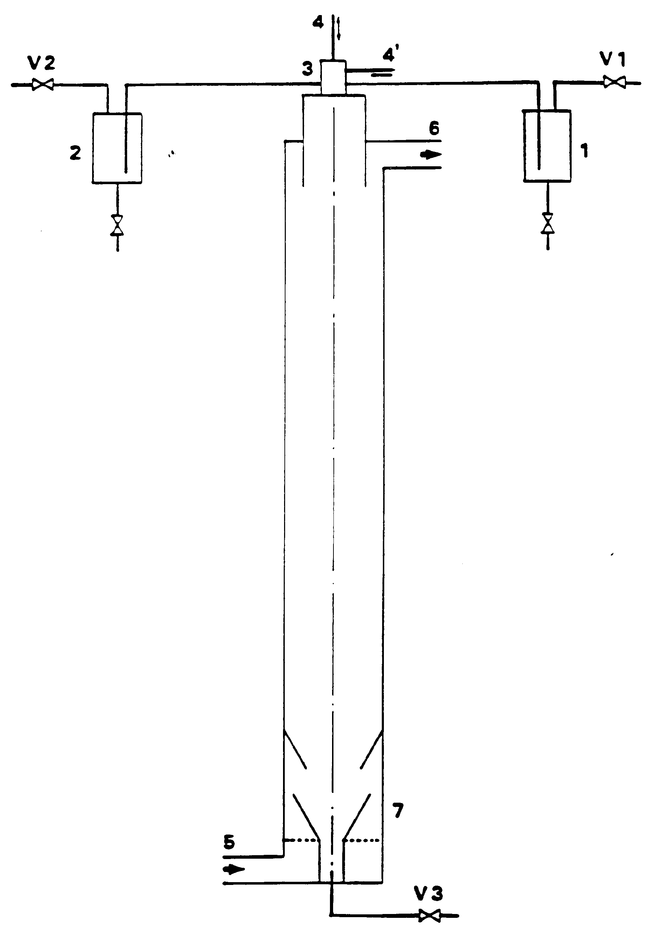

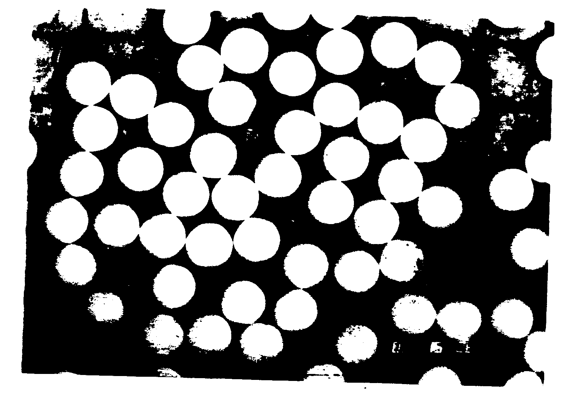

L'appareillage sera plus complètement décrit à l'aide de la figure 1 ci-annexée. Le principe actif présentant le phénomène de surfusion est introduit dans le récipient 1 et l'excipient dans le récipient 2 ou on introduit le mélange fondu, excipient principe actif dans chacun des deux récipients. Ces deux récipients sont maintenus sous atmosphère de gaz inerte. Au moyen de deux tubes, les liquides fondus sont amenés au-dessus d'une buse qui est maintenue dans une atmosphère non refroidie et qui est même éventuellement chauffée (3). La buse présente 1 à 24 perforations ou plus présentant de préférence un diamètre compris entre 50 et 600 µm (microns). La longueur de la perforation est de préférence comprise entre 0,5 et 10 fois le diamètre de celle-ci.

Cette buse est soumise à un système de vibration (4) électrique de haute fréquence (500 à 10000 hertz). L'air froid qui permet la solidification du principe actif et de l'excipient est introduit en bas de tour (5) et sort en-dessous de la buse (6) à une distance de préférence d'environ L/10 par rapport au sommet de la tour, L étant la hauteur de la tour.

Chaque orifice de la buse a de préférence une forme de cône inversé, la pointe du cône se trouvant dirigée vers la base de la tour ce qui permet d'obtenir un flux de liquide parfaitement laminaire.

La hauteur de la tour varie entre 1 mètre et une dixaine de mètres, la tour peut comporter au quart inférieur de sa hauteur une jupe perforée tronconique qui centre les perles dans le lit fluidisé.

Le lit fluidisé (7) éventuellement adjoint au bas de la tour est dans le cadre de la présente invention de préférence un lit. fluidisé en forme d'entonnoir équipé à sa base d'une grille de distribution permettant de minimiser l'adhérence sur les parois et de favoriser les chocs paroi-perles dans le but d'accroître la vitesse de solidification. L'adjonction de ce type d'appareil permet une solidification plus intense du mélange des constituants dans la bille, l'extérieur étant déjà solide à l'entrée dans le lit fluidisé.

Les billes ou perles obtenues par le procédé de la présente invention présentent une forme régulière et un diamètre compris entre 0,1 mm et 1,5 mm. La quantité de principe actif introduite varie de 5 à 95 % en poids et de préférence de 40 à 60 % en poids.

Ces perles peuvent être mises sous diverses formes pharmaceutiques telles que les sachets, les gelules, les comprimés.

Il est parfois utile d'ajouter, lors de leur mise en forme, des agents d'écoulement, des lubrifiants, des charges minérales (talc, silices, oxyde d'aluminium) pour éviter les phénomènes électrostatiques et des édulcorants (saccharinate, Aspartam) lors du conditionnement par exemple sous forme de sachets.

Les perles peuvent aussi, préalablement à leur formulation, être enrobées par une pellicule filmogène telle qu'un enrobage gastrorésistant à base notamment de résines cellulosiques ou acryliques et/ou revêtus d'une pellicule colorée.

La présente invention sera plus complètement décrite à l'aide des exemples suivants.

EXEMPLES

Les produits qui entrent dans la composition des perles sont indiqués dans le tableau 1.
Le tableau présente :
- la composition des mélanges,
- les conditions opératoires,
  i.e. le diamètre des trous,
      la fréquence des vibrations,
      la température de la buse.
Les vitesses superficielles de l'air de refroidissement et de l'azote de fluidisation sont 1,5 - 1,8 m/s et 0,3 - 1 m/s respectivement.

**EP 0 438 359 B1**

Procédure

Les couples sont fondus dans un réacteur agité puis transférés dans un ou les deux pots.

L'air de refroidissement est admis au débit désiré.

Le lit fluidisé est mis en service.

Lorsque la température de sottie de l'air est en régime, la vanne $V_1$ ou $V_2$ admet l'azote à la pression choisie et le liquide est transféré dans la buse.

Le réglage de la fréquence est fait au moyen d'un générateur en observant visuellement les perles avec un stroboscope (si nécessaire, l'air de refroidissement est aspiré à travers un lit de carboglace pour qu'il ait une température de 4 à 12°C avant d'entrer dans la tour).

Les perles sont récupérées dans le bas de la tour, elles présentent une dureté suffisante pour être mise en forme pharmaceutique.

Photo

La photo adjointe représente les perles obtenues à l'exemple 9, l'échelle entre deux lignes parallèles est de 1 mm.

Test de dissolution

On utilise l'appareil décrit dans la Pharmacopée Européenne, 2ème édition V 5.4. (1986) et l'USP XXI (711).

Méthode de dissolution Prilling :

+ prise d'essai 100 mg ou 200 mg en kétoprofène,
+ palette à 120 tours par minute,
+ milieux :
  . HCl 0,01 N          1 heure
     (8,6 ml HCl à 36 % p/p q.s.p 10 litres avec $H_2O$)
  . PH 4,5          2 heures
     68 g de dihydrogénophosphate de potassium q.s.p. 10 litres
  . PH 6,6          16 heures
     Ajouter x ml de soude 1N dans le milieu afin d'obtenir le PH souhaité :
     * x = 8 ml pour 500 ml de PH 4,5
     * x = 16 ml pour 1000 ml de PH 4,5
+ 500 ml à 37°C pour les prills dosés à 100 mg
  1000 ml à 37°C pour les prills dosés à 200 mg
+ lecture en continu à 260 nm avec des cuves de 1 mm.
  $E_{1cm}^{1\%}$ = 657 HClO, 01N
  $E_{1cm}^{1\%}$ = 661 PH 4,5
  $E_{1cm}^{1\%}$ = 659 PH 6,6

5

TABLEAU 1

| ESSAIS | COMPOSITION DES PERLES | | | PROTOCOLE OPERATOIRE | | | |
|---|---|---|---|---|---|---|---|
| | KETOPROFENE | PRECIROL | ACIDE STEARIQUE | DIAMETRE DE LA BUSE (mm) | FREQUENCE Hertz | TEMPERATURE DE LA BUSE °C | DIAMETRE MOYEN DES PERLES |
| 1 | 50 | 25 | 25 | 0,3 | 2880 | 95 | 600 µm |
| 2 | 60 | 40 | - | 0,3 | 3220 | 95 | 600 µm |
| 3 | 50 | 50 | - | 0,4 | 1400 | 95 | 800 µm |
| 4 | 50 | 50 | - | 0,3 | 4090 | 95 | 600 µm |
| 5 | 50 | 50 | - | 0,2 | 3580 | 100 | 450 µm |
| | | COMPRITOL | | | | | |
| 6 | 50 | 10 | 40 | 0,4 | 800 | 91 | 800 µm |
| 7 | 70 | 10 | 20 | 0,4 | 700 | 91 | 900 µm |
| 8 | 60 | 25 | 15 | 0,4 | 1350 | 92 | 800 µm |
| 9 | 67 | 13 | 20 | 0,4 | 1225 | 92 | 750 µm |
| | | ALCOOL CETYLIQUE | ETHYL CELLULOSE | | | | |
| 10 | 50 | 46,5 | 3,5 | 0,3 | 900 | 90 | 600 µm |
| | | STEARATE DE MAGNESIUM | ACIDE STEARIQUE | | | | |
| 11 | 60 | 20 | 20 | 0,4 | 800 | 101 | 800 µm |
| | MEPROBAMATE | | ACIDE STEARIQUE | | | | |
| 12 | 50 | 50 | 50 | 0,4 | 2400 | 95 | 750 µm |

**Revendications**

1. Procédé de préparation de perles à base d'un principe actif pharmaceutique dans lequel on mélange ledit

6

principe actif avec un excipient pharmaceutique favorisant la solidification du principe actif, on force le passage de la masse fondue à travers une buse qui subit une vibration, on laisse tomber les perles formées dans une tour à contre courant avec un gaz, on rassemble les perles formées dans le bas de la tour caractérisé en ce que le principe actif présente un point de cristallisation non défini et est utilisé à l'état fondu.

2. Procédé selon la revendication 1 caractérisé en ce que principe actif choisi est l'acide benzoyl-3 phényl-2 propionique.

3. Procédé selon la revendication 1 caractérisé en ce que l'excipient pharmaceutique favorisant la cristallisation du principe actif est choisi parmi les alcools gras, les acides gras et leurs sels, les esters du glycérol, les huiles hydrogénées, les polyols, les cires, les polyéthylènes glycols et leurs esters.

4. Procédé selon la revendication 2 et 3 caractérisé en ce que l'excipient pharmaceutique est un ester du glycérol choisi parmi le stéarate et le béhénate de glycérol.

5. Procédé selon l'une quelconque des revendications précédentes caractérisé en ce qu'on ajoute un additif de cristallisation choisi parmi les oxydes minéraux du silicium, du titane, les phosphates, les carbonates, le talc et les argiles.

6. Procédé selon l'une quelconque des revendications précédentes caractérisé en en qu'on ajoute un polymère soluble ou dispersible dans la masse fondue choisi parmi les dérivés cellulosiques, les résines acryliques, les acétates de polyvinyle, les résines de polyalkyléne, polylactique, de silicone.

7. Procédé selon l'une quelconque des revendications précédentes caractérisé en ce qu'on ajoute un agent tensioactif.

8. Perles susceptibles d'être obtenues selon l'une quelconque des revendications précédentes présentant un effet de libération retardée caractérisé en ce que le principe actif présente un point de cristallisation non défini et en ce que l'excipient est choisi parmi les acides gras, les esters de glycérol, les huiles hydrogénées, les cires et le polyoxyéthylèneglycols estérifiées.

9. Perles selon la revendication 8 caractérisées en ce qu'elles sont enrobées par un agent filmogène gastrorésistant et/ou colorant.

10. Formes pharmaceutiques caractérisés en ce qu'elles contiennent les perles obtenues par l'une quelconque des revendications précédentes.

11. Sachets, gelules selon la revendication 10 caractérisés en ce qu'ils contiennent les perles, des agents d'écoulement, des lubrifiants, des charges minérales et/ou des édulcorants.

**Patentansprüche**

1. Verfahren zur Herstellung von Kügelchen aus einem pharmazeutischen Wirkstoff, bei dem man besagten Wirkstoff mit einem pharmazeutischen Hilfsstoff vermischt, der die Verfestigung des Wirkstoffs unterstützt, bei dem man die geschmolzene Masse durch eine Düse preßt, die in eine Vibration versetzt wird, bei dem man die gebildeten Kügelchen in einem Turm mit einem Gasgegenstrom fallen läßt, bei dem man die gebildeten Kügelchen am Boden des Turms sammelt, dadurch gekennzeichnet, daß der Wirkstoff einen nicht definierten Kristallisationspunkt aufweist und im geschmolzenen Zustand eingesetzt wird.

2. Verfahren nach Anspruch 1, dadurch gekennzeichnet, daß der ausgewählte Wirkstoff 3-Benzoyl-2-phenylpropionsäure ist.

3. Verfahren nach Anspruch 1, dadurch gekennzeichnet, daß der pharmazeutische Hilfsstoff, der die Kristallisation des Wirkstoffs unterstützt, ausgewählt ist aus: den Fettalkoholen, den Fettsäuren und ihren Salzen, den Glycerinestern, den hydrierten Ölen, den Polyolen, den Wachsen, den Polyethylenglykolen und ihren Estern.

4. Verfahren nach Anspruch 2 und 3, dadurch gekennzeichnet, daß der pharmazeutische Hilfsstoff ein Glycerinester ist, ausgewählt aus dem Stearat und dem Behenat des Glycerins.

5. Verfahren nach einem beliebigen der vorhergehenden Ansprüche, das dadurch gekennzeichnet ist, daß man einen Kristallisationszusatz zugibt, ausgewählt aus: den Mineraloxiden des Siliciums, des Titan, den Phosphaten, den Carbonaten, dem Talkum und den Tonen.

6. Verfahren nach einem beliebigen der vorhergehenden Ansprüche, dadurch gekennzeichnet, daß man der geschmolzenen Masse ein lösliches oder dispergierbares Polymer zusetzt, ausgewählt aus den Cellulosederivaten, den Acrylharzen, den Polyvinylacetaten, den Polyalkylen-, Polymilchsäure- und Silikonharzen.

7. Verfahren nach einem beliebigen der vorhergehenden Ansprüche, dadurch gekennzeichnet, daß man ein Tensid zugibt.

8. Kügelchen, die nach einem beliebigen der vorhergehenden Ansprüche erhalten werden können und die eine verzögerte Freisetzung aufweisen, dadurch gekennzeichnet, daß der Wirkstoff einen nicht definierten Kristallisationspunkt aufweist und daß der Hilfstoff ausgewählt ist aus: den Fettsäuren, den Glycerinestern, den hydrierten Ölen, den Wachsen und den veresterten Polyethylenglykolen.

9. Kügelchen nach Anspruch 8, dadurch gekennzeichnet, daß sie mit einem magensaftresistenten Filmbildner und/oder einem Farbstoff überzogen sind.

10. Arzneiformen, dadurch gekennzeichnet, daß sie die Kügelchen enthalten, die nach irgendeinem der vorhergehenden Ansprüche erhalten wurden.

11. Beutel, Gelatinekapseln nach Anspruch 10, dadurch gekennzeichnet, daß sie die Kügelchen, Fließmittel, Gleitmittel, mineralische Füllmittel und/oder Süßstoffe enthalten.


## Claims

1. Process for the preparation of prills based on a pharmaceutical active substance, in which the said active substance is mixed with a pharmaceutical excipient promoting the solidification of the active substance, the melt is forced to pass through a nozzle which is subjected to a vibration, the prills formed are allowed to fall in a tower countercurrentwise to a gas, and the prills formed are collected in the bottom of the tower, characterized in that the active substance exhibits an indefinite crystallization point and is used in the molten state.

2. Process according to claim 1, characterized in that the chosen active substance is 2-(3-benzoylphenyl)propionic acid.

3. Process according to claim 1, characterized in that the pharmaceutical excipient promoting the crystallization of the active substance is chosen from fatty alcohols, fatty acids and their salts, glycerol esters, hydrogenated oils, polyols, waxes, polyethylene glycols and their esters.

4. Process according to claim 2 and 3, characterized in that the pharmaceutical excipient is a glycerol ester chosen from glycerol stearate and behenate.

5. Process according to any one of the preceding claims, characterized in that a crystallization additive chosen from inorganic oxides of silicon or of titanium, phosphates, carbonates, talc and clays is added.

6. Process according to any one of the preceding claims, characterized in that a polymer which is soluble or dispersible in the melt is added, chosen from cellulose derivatives, acrylic resins, polyvinyl acetates and polyalkylene, polylactic or silicone resins.

7. Process according to any one of the preceding claims, characterized in that a surface-active agent is added.

8. Prills capable of being obtained according to any one of the preceding claims, exhibiting a retarded release effect, characterized in that the active substance exhibits an indefinite crystallization point and in that the excipient is chosen from fatty acids, glycerol esters, hydrogenated oils, waxes and esterified polyoxyethylene glycols.

9. Prills according to claim 8, characterized in that they are coated with a gastroresistant and/or colouring film-forming agent.

10. Pharmaceutical forms characterized in that they contain the prills obtained using any one of the preceding claims.

11. Sachets and gelatin capsules according to claim 10, characterized in that they contain the prills, flow aids, lubricants, inorganic fillers and/or sweeteners.